(19) Europäisches Patentamt
European Patent Office
Office européen des brevets

(11) **EP 3 162 380 A1**

(12) **EUROPEAN PATENT APPLICATION**
published in accordance with Art. 153(4) EPC

(43) Date of publication:
**03.05.2017 Bulletin 2017/18**

(51) Int Cl.:
*A61K 45/00* (2006.01)     *A61K 31/436* (2006.01)
*A61K 31/661* (2006.01)     *A61P 17/00* (2006.01)

(21) Application number: **15811469.4**

(86) International application number:
**PCT/JP2015/069081**

(22) Date of filing: **25.06.2015**

(87) International publication number:
**WO 2015/199248 (30.12.2015 Gazette 2015/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **26.06.2014 JP 2014131788**

(71) Applicant: Osaka University
Suita-shi, Osaka 565-0871 (JP)

(72) Inventors:
• **KANEDA, Mari**
**Suita-shi**
**Osaka 565-0871 (JP)**
• **KATAYAMA, Ichiro**
**Suita-shi**
**Osaka 565-0871 (JP)**
• **MUROTA, Hiroyuki**
**Suita-shi**
**Osaka 565-0871 (JP)**
• **MATSUI, Saki**
**Suita-shi**
**Osaka 565-0871 (JP)**

(74) Representative: **Cabinet Plasseraud**
**66 rue de la Chaussée d'Antin**
**75440 Paris Cedex 09 (FR)**

(54) **ANTIPERSPIRANT AGENT**

(57) The present invention provides an antiperspirant which can effectively and safely control perspiration. The antiperspirant in accordance with an aspect of the present invention contains a mTOR inhibitor. The mTOR inhibitor can be rapamycin, a rapamycin derivative, or the like, and the antiperspirant can be used against hyperhidrosis and the like.

FIG. 13

EP 3 162 380 A1

## Description

Technical Field

The present invention relates to an antiperspirant and an agent for treating hyperhidrosis.

Background Art

The number of patients who suffer from a disease involving perspiration (e.g., hyperhidrosis) is fairly large, and it is extremely important for those patients to develop a technique to adjust perspiration.

For example, according to a questionnaire survey answered by 5807 respondents ranging from age 5 to age 64, 810 respondents answered that they had subjective symptoms of hyperhidrosis. That is, 14% of the subjects of survey have subjective symptoms of hyperhidrosis. In a case where the ratio is converted to the entire population of Japan, approximately 17,320,000 people have subjective symptoms of hyperhidrosis. Note that, according to results of the questionnaire survey, it is reported that ratios of respondents who have subjective symptoms of hyperhidrosis are substantially equal to each other among almost all age groups.

Moreover, there are various kinds of hyperhidrosis such as severe primary palmoplantar hyperhidrosis, intractable severe primary palmoplantar hyperhidrosis, palmar hyperhidrosis, plantar hyperhidrosis, and maschalyperidrosis.

According to a certain survey, the followings are reported: that is, approximately 0.64% of the entire population of Japan (i.e., approximately 801,200 people) suffer from severe primary palmoplantar hyperhidrosis, and approximately 5.6% of patients who suffer from severe primary palmoplantar hyperhidrosis (i.e., approximately 45,000 people) suffer from intractable severe primary palmoplantar hyperhidrosis.

Moreover, according to a certain survey, the followings are reported: that is, an average age of developing palmar hyperhidrosis is 13.3 and approximately 5.5% of people of that age develop palmar hyperhidrosis; an average age of developing plantar hyperhidrosis is 15.5 and approximately 2.7% of people of that age develop plantar hyperhidrosis; and an average age of developing maschalyperidrosis is 19.4 and approximately 5.7% of people of that age develop maschalyperidrosis.

Moreover, according to the survey, the followings are reported: that is, in a case where patients suffering from palmar hyperhidrosis, plantar hyperhidrosis, and maschalyperidrosis are classified in accordance with severity of symptoms, 12.2% of the patients are classified into a severe symptom, 34.6% of the patients are classified into a moderate symptom, and 53.2% of the patients are classified into a mild symptom.

As such, the number of patients who suffer from the disease involving perspiration (e.g., hyperhidrosis) is fairly large, and there is an urgent need for development of a technique to control perspiration.

Under the circumstances, until now, various techniques to control perspiration have been developed (see, for example, Non-Patent Literature 1). For example, as techniques to control perspiration, there are techniques such as botulinus toxin local injection therapy and sympathectomy.

Citation List

Non-patent Literature

Non-Patent Literature 1: Guideline of The Japanese Dermatological Association, The Japanese journal of dermatology: 120(8), 1607-1625, 2010

Summary of Invention

Technical Problem

However, the above conventional techniques have a problem that perspiration cannot be effectively and safely controlled.

For example, the botulinus toxin local injection therapy which is a method of locally injecting botulinus toxin into a living body has the following problems: that is, (a) perspiration is controlled only in a part of the body, in other words, perspiration on the entire body cannot be controlled, (b) there is a possibility that the living body would be adversely affected by the injection of botulinus toxin, (c) pain caused by the injection and decrease in muscle force of the hand, and (d) it is necessary to externally apply a local anesthetic, to cool with an ice pack, to administer an intravenous injection of an anesthetic, or the like before the injection, in order to control the pain caused by the injection.

Moreover, the sympathectomy which is a method of physically cutting a part of a sympathetic nerve has the following problems: that is, (e) there is a possibility that the living body is adversely affected by the cutting of the sympathetic nerve, (f) it is difficult to accurately inhibit perspiration in an intended region because a region of the body which region is governed by a nerve varies depending on individuals, (g) the sympathectomy is a therapeutic method of high invasiveness, and nerve block is irreversible, and (h) the sympathectomy is complicated with compensatory perspiration.

The present invention is accomplished in view of the conventional problems, and its object is to provide an antiperspirant which can effectively and safely control perspiration.

Solution to Problem

As a result of diligent study in view of the problems, the inventors of the present invention have found that perspiration is facilitated in accordance with activation of mammalian target of rapamycin (mTOR), conversely, perspiration is inhibited by inactivation of mTOR. Based on this finding, the inventors have accomplished the present invention.

In order to attain the object, an antiperspirant in accordance with an aspect of the present invention contains a mTOR inhibitor.

An agent for treating hyperhidrosis in accordance with an aspect of the present invention contains a mTOR inhibitor.

Advantageous Effects of Invention

The present invention can bring about an effect of inhibiting perspiration.

The antiperspirant (in particular, external medicine) in accordance with an aspect of the present invention brings about an effect of safely inhibiting perspiration without adversely affecting (e.g., causing a side effect in) the living body.

Specifically, the antiperspirant in accordance with an aspect of the present invention transiently inhibits perspiration and then restores perspiration performance of the living body. Therefore, the present invention can safely inhibit perspiration without adversely affecting the living body.

The antiperspirant in accordance with an aspect of the present invention brings about an effect of not only locally inhibiting perspiration of the living body but also inhibiting perspiration on the whole body of the living body. For example, in a case where the present invention is used as an external medicine, it is possible to locally inhibit perspiration of the living body. Further, in a case where the present invention is used as an internal medicine or an injectable medicine, it is possible to inhibit perspiration on the whole body of the living body.

In particular, the external medicine is suitable because the external medicine can be easily used, can adequately bring about an effect at an intended site, and can be used for a long time without worry of a side effect in the whole body.

Brief Description of Drawings

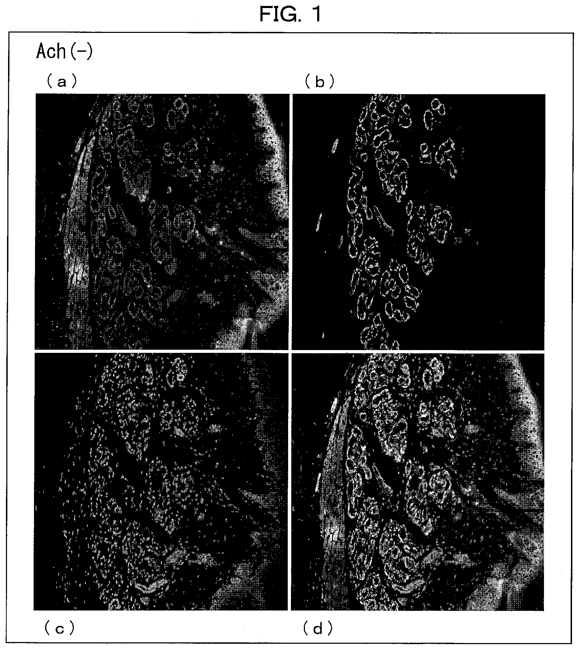

Each of (a) through (d) of Fig. 1 is a stained photograph showing a state of a mTOR to which no sudorific stimulus is given in Example.

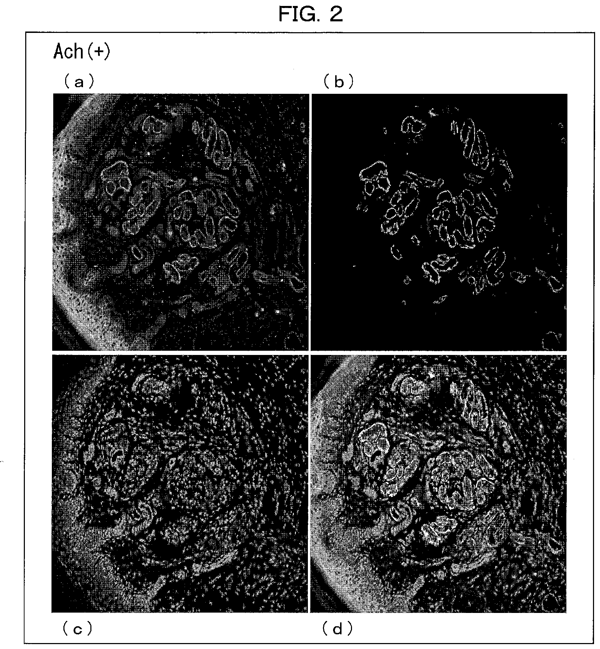

Each of (a) through (d) of Fig. 2 is a stained photograph showing a state of a mTOR to which a sudorific stimulus has been given in Example.

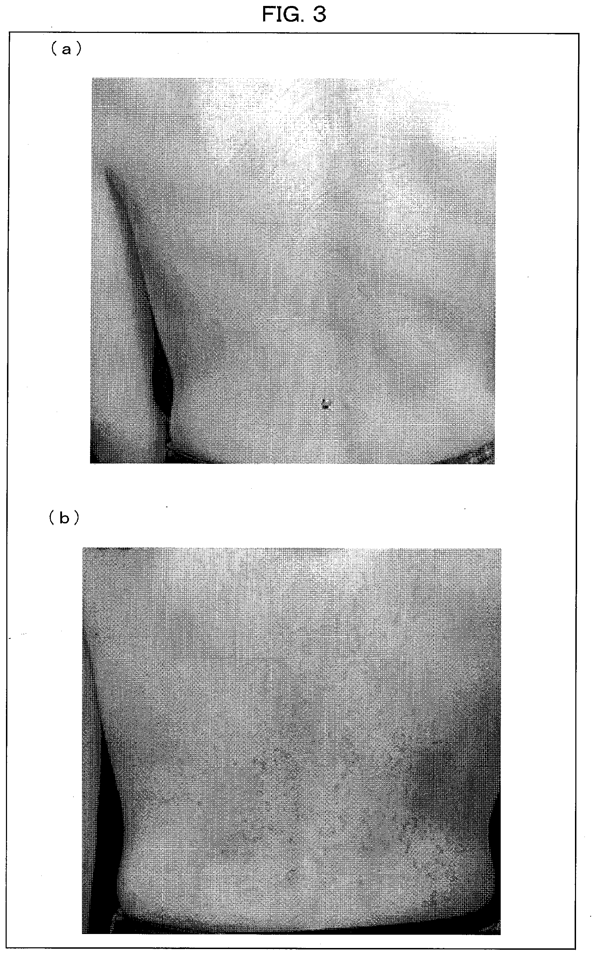

(a) of Fig. 3 is a photograph showing a state of a skin before a mTOR inhibitor is orally administered in Example, and (b) of Fig. 3 is a photograph showing a state of the skin after the mTOR inhibitor is orally administered in Example.

Fig. 4 is a graph showing change in amount of sweating in a case where the mTOR inhibitor has been orally administered in Example.

Fig. 5 is a photograph showing an influence exerted on perspiration by administration of the mTOR inhibitor (external application, single administration) in Example.

Fig. 6 is a graph showing an influence exerted on perspiration by administration of the mTOR inhibitor (external application, single administration) in Example.

Fig. 7 is a graph showing an influence exerted on perspiration by administration of the mTOR inhibitor (external application, sequential administration) in Example.

Fig. 8 is a graph showing an influence exerted on perspiration by administration of the mTOR inhibitor (internal application, sequential administration) in Example.

Fig. 9 is a graph showing an influence exerted on perspiration by administration and withdrawal of the mTOR inhibitor (internal application, withdrawal of administration after sequential administration) in Example.

Fig. 10 is a graph showing an effect exerted on an amount of sweating (of healthy subject) by a gel preparation which contains rapamycin in Example.

Fig. 11 is a graph showing an effect exerted on an amount of sweating (of healthy subject) by a gel preparation which contains rapamycin in Example.

Fig. 12 is a graph showing an effect exerted on an amount of sweating (of healthy subject) by a gel preparation which contains rapamycin in Example.

Fig. 13 is a graph showing an effect exerted on an amount of sweating (of hyperhidrosis patient subject) by a gel preparation which contains rapamycin in Example.

Fig. 14 is a graph showing an effect exerted on an amount of sweating (of hyperhidrosis patient subject) by a gel preparation which contains rapamycin in Example.

Description of Embodiments

The present invention is not limited to the embodiments described below, and embodiments can be altered by a skilled person in the art within the scope of the claims. An embodiment or an example derived from a proper combination of technical means disclosed in respective different embodiments and Examples is also encompassed in the technical scope of the present invention.

In this specification, a description "A to B" means "A or more (higher) and B or less (lower)".

The antiperspirant in accordance with an aspect of the present invention contains a mTOR inhibitor as an active ingredient which brings about a perspiration inhibitory effect.

The mTOR inhibitor is not limited, provided that the mTOR inhibitor can lower activity of a mammalian target of rapamycin (mTOR) in a living body (e.g., cell, tissue, individual, and the like).

For example, the mTOR inhibitor can be an inhibitor which interferes with transcription and/or translation of a mTOR gene, can be an inhibitor which interferes with activation of a mTOR protein, or can be an inhibitor which facilitates decomposition of a mTOR protein. Of course, the mTOR inhibitor can be an inhibitor which lowers activity of a mTOR by a function other than the above described ones.

Examples of the mTOR inhibitor encompass rapamycin, a rapamycin derivative, and a mTOR kinase inhibitor.

Among the above exemplified mTOR inhibitors, the rapamycin and the rapamycin derivative have already been used to treat other diseases, and thus safety of those has been confirmed in clinical practice. Therefore, by using any of these mTOR inhibitors, it is possible to provide an antiperspirant which has higher safety.

The rapamycin derivative is not limited to a particular one, and examples of the rapamycin derivative encompass everolimus, temsirolimus, ridaforolimus, and zotarolimus.

Among the mTOR inhibitors, rapamycin and everolimus are preferable because these are chemical agents which have been used from long ago and are inexpensive.

The mTOR kinase inhibitor is not limited to a particular one, and examples of the mTOR kinase inhibitor encompass MLN-0128 and CC-223. Of course, mTOR kinase inhibitors other than those can also be used.

The antiperspirant in accordance with an aspect of the present invention can be used to treat various symptoms involving perspiration. The antiperspirant in accordance with an aspect of the present invention can be used to treat, for example, hyperhidrosis, miliaria, eccrine hidrocystoma, or dysidrosis (pompholyx).

Hyperhidrosis is classified into generalized hyperhidrosis in which perspiration on the whole body increases and focal hyperhidrosis in which an amount of sweating increases only in a part of the body. The generalized hyperhidrosis includes primary generalized hyperhidrosis which develops for no cause and secondary generalized hyperhidrosis which develops as complication of another disease. Meanwhile, the focal hyperhidrosis also includes primary focal hyperhidrosis and secondary focal hyperhidrosis. The antiperspirant in accordance with an aspect of the present invention can be used to treat any of those kinds of hyperhidrosis. Specific examples of hyperhidrosis encompass various kinds of primary hyperhidrosis such as primary maschalyperidrosis, primary palmar hyperhidrosis, and primary plantar hyperhidrosis.

Among the antiperspirants of the present invention, the external medicine can be adequately applied locally to the body, and therefore the external medicine is particularly preferably used to treat local hyperhidrosis such as palmoplantar hyperhidrosis or all kinds of hyperhidrosis which show local symptoms such as dysidrosis (pompholyx).

The antiperspirant in accordance with an aspect of the present invention can be used with respect to not only humans but also non-human animals. Examples of the non-human animals encompass mammals except human. Examples of the mammals except human encompass artiodactyls such as cattle, wild boar, pig, sheep, and goat; perissodactyls such as horse; rodents such as mouse, rat, hamster, and squirrel; Lagomorpha such as rabbit; and carnivores such as dog, cat, and ferret. The non-human animals are not limited to domestic animals or companion animals (pets) and can be wild animals.

A route of administration of the antiperspirant in accordance with an aspect of the present invention is not limited, and can be any of routes of administration such as percutaneous, oral, enteral, intravenous, and transmucosal. Therefore, the antiperspirant in accordance with an aspect of the present invention can be in any of forms such as an internal medicine, an external medicine, an injectable medicine, a suppository, and an inhalant. The antiperspirant in accordance with an aspect of the present invention is preferably in a form of external medicine, internal medicine, or injectable medicine. More preferably, the antiperspirant in accordance with an aspect of the present invention is in the form of external medicine.

In any of the forms, it is possible to effectively inhibit perspiration. However, in the form of external medicine, it is easy to locally inhibit perspiration of the living body and, in the form of internal medicine or injectable medicine, it is easy to inhibit perspiration on the whole body of the living body. From the viewpoint of easiness and safety, the external medicine is particularly preferable.

In the following descriptions, first, a concrete configuration will be discussed in which the antiperspirant is in the form of external medicine, and then, a concrete configuration in which the antiperspirant is in the form of internal medicine will be discussed.

(A) External medicine

The following description will discuss an antiperspirant in the form of external medicine.

Examples of the external medicine encompass an ointment, a gel preparation, a patch, a poultice, a liniment, a lotion, a cream, and the like.

For example, i) the gel preparation can be prepared by causing a solution containing a mTOR inhibitor to gelate. Moreover, ii) an ointment containing a mTOR inhibitor can be prepared with a known method. Each of the patch, the poultice, the liniment, the lotion, and the cream can be prepared in accordance with a known method.

By topically administering the antiperspirant in such a form of external medicine, it is possible to enhance a perspiration inhibitory effect by the mTOR inhibitor.

Moreover, the antiperspirant thus prepared can bring about an intended effect with a small amount of the mTOR inhibitor. It is therefore possible to further reduce a side effect.

Note that the medicine can be absorbed better in the form of gel preparation than in the form of ointment, and therefore the effects are effectively brought about in the form of gel preparation. In view of this, the gel preparation is more preferable than the ointment.

The following description will discuss a specific configuration of the gel preparation and a specific configuration of the ointment.

(A-1) Gel preparation

As above described, the antiperspirant in accordance with an aspect of the present invention can be a gel preparation which is obtained by causing a solution containing a mTOR inhibitor to gelate.

In a case where a solution containing a mTOR inhibitor is caused to gelate, the solution is caused to gelate with use of a gelation inducer. In a case where, for example, Carbopol (Registered Trademark) 934NF is used as the gelation inducer, Carbopol (Registered Trademark) 934NF is added to the solution, and further, pH of the solution is adjusted to neutral with a pH adjuster such as trishydroxymethylaminomethane. Thus, it is possible to induce gelation of the solution.

Examples of a solvent in the solution encompass isopropanol, ethanol, and propylene carbonate. Among those solvents, isopropanol and ethanol are more preferable. With the configuration, it is possible to provide a gel preparation which is further effective.

An amount of the solvent contained in the gel preparation is not limited in particular, provided that the mTOR inhibitor can be sufficiently dissolved. For example, a weight of the solvent can be 100 times to 300 times greater than a weight of the mTOR inhibitor, or can be 120 times to 250 times greater than the weight of the mTOR inhibitor.

An amount of the gelation inducer contained in the gel preparation is not limited in particular, provided that the amount is sufficient for the solution containing the mTOR inhibitor to gelate.

For example, in a case where a gelation inducer such as Carbopol (Registered Trademark) and a pH adjuster (such as trishydroxymethylaminomethane) are used as components for inducing gelation, the amount of the gelation inducer can be, for example, 1.6% by weight, and the amount of the pH adjuster can be, for example, 0.4% by weight, 0.6% by weight, or 0.8% by weight, relative to a total weight of the external medicine. Of course, the present invention is not limited to these ratios.

The gel preparation can contain other components, in addition to the mTOR inhibitor. Examples of the other components encompass a water-soluble polymer, a pH adjuster, water, and efficacious components other than the mTOR inhibitor.

Examples of the water-soluble polymer encompass polyethylene glycol, starch, methyl cellulose, hydroxypropyl cellulose (HPC), polyvinyl alcohol, polyvinyl methyl ether, polyvinyl pyrrolidone, and the like.

In a case where the gel preparation contains hydroxypropyl cellulose, it is possible to improve adhesion of the gel preparation. That is, the gel preparation becomes difficult to peel off from the skin.

An amount of the other components contained in the gel preparation is not limited in particular and can be, for example, not more than 50% by weight, not more than 40% by weight, not more than 30% by weight, not more than 20% by weight, or not more than 10% by weight, relative to a total weight of the gel preparation.

An amount of the mTOR inhibitor contained in the gel preparation is not limited in particular and is, for example, preferably not less than 0.001% by weight and not more than 2.0% by weight, more preferably not less than 0.01% by weight and not more than 1.0% by weight, more preferably not less than 0.1% by weight and not more than 0.9% by weight, and most preferably not less than 0.2% by weight and not more than 0.8% by weight, relative to the total weight of the gel preparation. Moreover, the amount of the mTOR inhibitor contained in the gel preparation can be, for example, not less than 0.02% by weight and not more than 0.9% by weight, not less than 0.03% by weight and not more than 0.8% by weight, not less than 0.04% by weight and not more than 0.7% by weight, not less than 0.05% by weight and not more than 0.6% by weight, not less than 0.06% by weight and not more than 0.5% by weight, not less than 0.07% by weight and not more than 0.4% by weight, not less than 0.08% by weight and not more than 0.3% by weight, not less than 0.09% by weight and not more than 0.25% by weight, and not less than 0.1% by weight and not more than 0.2% by weight, relative to the total weight of the gel preparation. Note that a lower limit of these ranges can be 0.05% by weight. Moreover, the amount of the mTOR inhibitor can be not less than 0.4% by weight and not more than 0.8% by weight.

An upper limit of the amount of the mTOR inhibitor contained in the gel preparation is not limited to a particular one and is, for example, preferably 0.8% by weight, and more preferably 0.4% by weight.

With the above described range of the amount, it is possible to effectively inhibit perspiration and to enhance safety of the antiperspirant.

An application amount of the mTOR inhibitor per unit surface area of the living body per day is not limited in particular. In a case where the mTOR inhibitor is rapamycin or a rapamycin derivative, the application amount of the mTOR inhibitor is, for example, 0.0001 mg/cm$^2$ or more and 2 mg/cm$^2$ or less, preferably 0.0002 mg/cm$^2$ or more and 1 mg/cm$^2$ or less, more preferably 0.0005 mg/cm$^2$ or more and 0.5 mg/cm$^2$ or less, more preferably 0.001 mg/cm$^2$ or more and 0.05 mg/cm$^2$ or less, and more preferably 0.00125 mg/cm$^2$ or more and 0.04 mg/cm$^2$ or less. Note that the mTOR inhibitor in the above amount can be applied once a day or can be applied in several doses in a day. In other words, the gel preparation in accordance with an aspect of the present invention is preferably a gel preparation which can be applied in the above described application amount.

It is preferable to adjust a concentration of the gel preparation in a suitable formulation and the number of administration depending on ages, administration sites, thicknesses of the skin, and the like. In a case where the mTOR inhibitor is rapamycin or a rapamycin derivative, for example, the gel preparation at a concentration of 0.05% to 0.2% can be applied once to three times a day to a thin skin, an armpit, or the like of a small child. Alternatively, the gel preparation at a concentration of 0.2% to 0.8% can be applied once to twice a day to a palm, a sole, or the like.

The following description will discuss a further concrete composition example of the gel preparation. The present invention is not limited to the composition. Note that, with the configuration described below, it is possible to further enhance a perspiration inhibitory effect by the mTOR inhibitor. Moreover, with the configuration described below, it is possible to bring about an intended effect with a smaller amount of the mTOR inhibitor, and it is therefore possible to further prevent occurrence of a side effect.

The gel preparation can contain Carbopol (Registered Trademark) 934NF, water, alcohol (e.g., ethanol or isopropanol (preferably, ethanol)), and trishydroxymethylaminomethane, in addition to the mTOR inhibitor.

In this case, a weight ratio of the mTOR inhibitor, Carbopol (Registered Trademark) 934NF, water, alcohol, and trishydroxymethylaminomethane can be 0.5-2 : 16 : 490 : 450-500 : 6.

The above ratio of the mTOR inhibitor, Carbopol (Registered Trademark) 934NF, water, alcohol, and trishydroxymethylaminomethane can be 0.5-2 : 16 : 490 : 480-490 : 6, or can be 2 : 16 : 490 : 486 : 6.

(A-2) Ointment

As early described, the antiperspirant in accordance with an aspect of the present invention can be an ointment which can be obtained by adding the mTOR inhibitor to an unguent. Examples of a base material encompass waxes (e.g., natural waxes such as white beeswax, lanolin, carnauba wax, and spermaceti wax; mineral waxes such as montan wax; and synthetic waxes); paraffins (e.g., liquid paraffin, solid paraffin, and the like); petrolatums (e.g., white petrolatum, yellow petrolatum, and the like); and the like.

An amount of the base material is not limited in particular and can be, for example, not less than 10% by weight, not less than 20% by weight, not less than 30% by weight, not less than 40% by weight, not less than 50% by weight, not less than 60% by weight, not less than 70% by weight, not less than 80% by weight, or not less than 90% by weight, relative to a total weight of the ointment.

The ointment can contain other components in addition to the mTOR inhibitor. The other components can be those exemplified in the above (A-1). Note that the details of those components have already been described, and therefore the detailed descriptions are omitted here.

The ointment can contain propylene carbonate, solid paraffin, and white petrolatum, in addition to the mTOR inhibitor. The ointment can also contain liquid paraffin, in addition to propylene carbonate, solid paraffin, and white petrolatum. The ointment can also contain white beeswax, in addition to propylene carbonate, solid paraffin, white petrolatum, and liquid paraffin.

In this case, a weight ratio of the mTOR inhibitor, propylene carbonate, solid paraffin, white petrolatum, liquid paraffin, and white beeswax can be 0.3-10 : 50-59.4 : 30-45 : 895 : 0-10 : 0-5. Note however that, according to the above weight ratio, a sum of (i) the weight ratio of the active ingredient, (ii) the weight ratio of propylene carbonate, (iii) the weight ratio of solid paraffin, and (iv) the weight ratio of liquid paraffin is 105.

More specifically, the weight ratio of the mTOR inhibitor, propylene carbonate, solid paraffin, white petrolatum, liquid paraffin, and white beeswax can be also 2 : 58 : 30 : 895 : 10 : 5 (hereinafter, referred to as a ratio 3). Further, the weight ratio of the mTOR inhibitor, propylene carbonate, solid paraffin, white petrolatum, liquid paraffin and white beeswax can be 2 : 58 : 45 : 895 : 0 : 0 (hereinafter, referred to as a ratio 4). Furthermore, the weight ratio of the mTOR inhibitor, propylene carbonate, solid paraffin, white petrolatum, liquid paraffin and white beeswax can be 2 : 58 : 35 : 895 : 10 : 0 (hereinafter, referred to as a ratio 5).

In a case where the ratio 3 and the ratio 4 are compared with each other, the external medicine produced with the ratio 3 is more transparent and includes less water in its surface than the external medicine produced with the ratio 4.

In a case where the ratio 3 and the ratio 5 are compared with each other, the external medicine produced with the ratio 3 is smoother and includes less water in its surface than the external medicine produced with the ratio 5.

An amount of the mTOR inhibitor contained in the ointment is not limited in particular and is, for example, preferably not less than 0.001% by weight and not more than 2.0% by weight, more preferably not less than 0.01% by weight and not more than 1.0% by weight, more preferably not less than 0.1% by weight and not more than 0.9% by weight, and most preferably not less than 0.2% by weight and not more than 0.8% by weight, relative to the total weight of the ointment. Moreover, the amount of the mTOR inhibitor contained in the ointment can be, for example, not less than 0.02% by weight and not more than 0.9% by weight, not less than 0.03% by weight and not more than 0.8% by weight, not less than 0.04% by weight and not more than 0.7% by weight, not less than 0.05% by weight and not more than 0.6% by weight, not less than 0.06% by weight and not more than 0.5% by weight, not less than 0.07% by weight and not more than 0.4% by weight, not less than 0.08% by weight and not more than 0.3% by weight, not less than 0.09% by weight and not more than 0.25% by weight, and not less than 0.1 % by weight and not more than 0.2% by weight, relative to the total weight of the ointment. Note that a lower limit of these ranges can be 0.05% by weight. Moreover, the amount of the mTOR inhibitor can be not less than 0.4% by weight and not more than 0.8% by weight.

With the above described amount, it is possible to effectively inhibit perspiration.

An upper limit of the amount of the mTOR inhibitor contained in the ointment is not limited to a particular one and is, for example, preferably 0.8% by weight, and more preferably 0.4% by weight.

With the above upper limit, it is possible to further prevent a local side effect of the mTOR inhibitor.

An application amount of the mTOR inhibitor per unit surface area of the living body per day is not limited in particular. In a case where the mTOR inhibitor is rapamycin or a rapamycin derivative, the application amount of the mTOR inhibitor is, for example, 0.0001 mg/cm$^2$ or more and 2 mg/cm$^2$ or less, preferably 0.0002 mg/cm$^2$ or more and 1 mg/cm$^2$ or less, more preferably 0.0005 mg/cm$^2$ or more and 0.5 mg/cm$^2$ or less, more preferably 0.001 mg/cm$^2$ or more and 0.05 mg/cm$^2$ or less, and more preferably 0.00125 mg/cm$^2$ or more and 0.04 mg/cm$^2$ or less. Note that the mTOR inhibitor in the above amount can be applied once a day or can be applied in several doses in a day. In other words, the ointment in accordance with an aspect of the present invention is preferably an ointment which can be applied in the above described application amount.

It is preferable to adjust a concentration of the ointment in a suitable formulation and the number of administration depending on ages, administration sites, thicknesses of the skin, and the like. In a case where the mTOR inhibitor is rapamycin or a rapamycin derivative, for example, the ointment at a concentration of 0.05% to 0.2% can be applied once to three times a day to a thin skin, an armpit, or the like of a small child. Alternatively, the ointment at a concentration of 0.2% to 0.8% can be applied once to twice a day to a palm, a sole, or the like.

The ointment can be produced with a known method. The following description will discuss an example of the production method.

For example, a solution in which the mTOR inhibitor is dissolved is added to a base material and stirred with use of a planetary centrifugal mixer (e.g., manufactured by THINKY CORPORATION) or a homomixer (e.g., manufactured by PRIMIX Corporation). In a case where the planetary centrifugal mixer is used, for example, the mixture can be stirred at 2000 rpm for 1 minute, then stirred at 1000 rpm for 5 minutes, and then stirred at 500 rpm for 3 minutes. In a case where the homomixer is used, for example, conditions of stirring can be set as appropriate by a person skilled in the art based on an instruction manual or the like attached to the homomixer. With use of the homomixer, it is possible to prepare a large amount of the ointment.

Note that, in a case where the base material is solid at a room temperature, the base material can be heated up so as to become liquid and then mixed with the solution in which the mTOR inhibitor is dissolved. For example, various components (such as waxes, paraffins, petrolatums) which are solid at a room temperature are melted by being heated at a temperature (e.g., 70°C) that is not lower than a melting point, and are then mixed and stirred with the solution in which the mTOR inhibitor is dissolved. Subsequently, the mixture is cooled to around a room temperature (e.g., 40°C) while being stirred, and thus the ointment can be produced.

In a case where those other components are contained in the ointment, it is possible that the mTOR inhibitor and those other components are dissolved in the solvent for dissolving the mTOR inhibitor, and then a resultant solution and the base material are mixed.

(B) Internal medicine

The following description will discuss an antiperspirant which is in the form of internal medicine.

In a case of the antiperspirant which is in the form of internal medicine, the antiperspirant can be produced by mixing the mTOR inhibitor with a component which is used in a known internal medicine.

An amount of the mTOR inhibitor that is contained in one dose of the internal medicine is not limited in particular, and can be set as appropriate depending on administration subjects.

For example, a dosage of the mTOR inhibitor to an adult per day is preferably 0.001 mg or more and 10 mg or less, preferably 0.01 mg or more and 9 mg or less, preferably 0.05 mg or more and 8 mg or less, preferably 0.1 mg or more and 7 mg or less, preferably 0.1 mg or more and 6 mg or less, more preferably 0.1 mg or more and 5 mg or less, preferably 0.1 mg or more and 4 mg or less, preferably 0.1 mg or more and 3 mg or less, more preferably 0.1 mg or more and 2 mg or less, and more preferably 0.1 mg or more and 1 mg or less.

With the above described amount, it is possible to effectively inhibit perspiration.

With the above described upper limit, it is possible to further prevent a side effect caused by the mTOR inhibitor.

A form of the internal medicine is not limited and can be, for example, tablet, capsule, powder, dispersing agent, syrup, or the like.

(C) Injectable medicine

The following description will discuss an antiperspirant which is in the form of injectable medicine. Note that the injectable medicine encompasses a drip.

In a case of the antiperspirant which is in the form of injectable medicine, the antiperspirant can be produced by mixing the mTOR inhibitor with a component which is used in a known injectable medicine.

An amount of the mTOR inhibitor that is contained in one dose of the injectable medicine is not limited in particular, and can be set as appropriate depending on administration subjects.

For example, a dosage of the mTOR inhibitor to an adult per day is preferably 0.001 mg or more and 10 mg or less, preferably 0.01 mg or more and 9 mg or less, preferably 0.05 mg or more and 8 mg or less, preferably 0.1 mg or more and 7 mg or less, preferably 0.1 mg or more and 6 mg or less, more preferably 0.1 mg or more and 5 mg or less, preferably 0.1 mg or more and 4 mg or less, preferably 0.1 mg or more and 3 mg or less, more preferably 0.1 mg or more and 2 mg or less, and more preferably 0.1 mg or more and 1 mg or less.

With the above described amount, it is possible to effectively inhibit perspiration.

With the above described upper limit, it is possible to further prevent a side effect caused by the mTOR inhibitor.

The injectable medicine can contain a sufficient amount of salt (e.g., sodium chloride, potassium chloride, or the like), glucose, or the like so as to be isotonic with blood.

The antiperspirant in accordance with an aspect of the present invention can be used as an agent for treating hyperhidrosis. The agent for treating hyperhidrosis in accordance with an aspect of the present invention includes a mTOR inhibitor. The descriptions regarding the antiperspirant in accordance with an aspect of the present invention is also applicable to the agent for treating hyperhidrosis in accordance with an aspect of the present invention.

Note that a method for inhibiting perspiration with use of the antiperspirant of the present invention and a method for treating hyperhidrosis with use of the antiperspirant of the present invention are also encompassed in the scope of the present invention. The method for inhibiting perspiration and the method for treating hyperhidrosis includes an administration step of administering the antiperspirant of the present invention to a patient (human or non-human animal) through the above described route of administration with the above described dosage and the above described number of administration. That is, the method for inhibiting perspiration and the method for treating hyperhidrosis includes an administration step of administering the antiperspirant of the present invention to a patient. A method for administering the antiperspirant in the administration step is not limited in particular, and the antiperspirant can be administered to a whole body with a method such as oral administration and intravascular (intravenous or intraarterial) administration, or can be topically administered by percutaneous administration in which the antiperspirant of the present invention is applied to a skin. For example, in a case where the antiperspirant of the present invention which is in the form of internal medicine, injectable medicine, or the like is administered by whole body administration, it is possible to inhibit perspiration on a whole body of a living body. In particular, in a case where the antiperspirant of the present invention in the form of external medicine is topically administered, it is possible to adequately bring about an effect at an intended site, and there is no worry of a side effect in the whole body. Therefore, the administration step is preferably a step of applying the antiperspirant of the present invention to an affected part of a patient.

In order to attain the object, the antiperspirant in accordance with an aspect of the present invention contains a mTOR inhibitor.

According to the antiperspirant in accordance with an aspect of the present invention, the mTOR inhibitor is preferably rapamycin or a rapamycin derivative.

According to the antiperspirant in accordance with an aspect of the present invention, the rapamycin derivative is preferably everolimus, temsirolimus, ridaforolimus, or zotarolimus.

The antiperspirant in accordance with an aspect of the present invention is preferably an external medicine, an internal medicine, or an injectable medicine.

The antiperspirant in accordance with an aspect of the present invention is more preferably an external medicine.

The antiperspirant in accordance with an aspect of the present invention is preferably used to treat hyperhidrosis, miliaria, eccrine hidrocystoma, or dysidrosis (pompholyx).

The agent for treating hyperhidrosis in accordance with an aspect of the present invention includes a mTOR inhibitor.

Example

<1. Analysis on mechanism to adjust perspiration>

In order to analyze a mechanism to adjust perspiration, molecules whose state changes in a case where a sudorific stimulus (acetylcholine) has been given to a mouse were inspected. The following description will discuss a test method and test results.

C57BL6J mice each of which was 7 week-old and female were bought from Clea (Osaka, Japan). During the test, the mice were bred in an aseptic animal feeding facility. In this Example and the later described Examples, the animals were managed in conformity to a guideline of Osaka University.

General anesthesia was applied to the mouse by intraabdominally injecting, to the mouse, 0.3 mg of Domitor, 5 mg of Betorufaru, a 4 mg of Dormicum per 1 kg of a body weight of the mouse.

Next, acetylcholine (pilocarpine hydrochloride' Junsei Chemical, Co., Ltd. Tokyo, Japan) which served as a sudorific stimulus was dissolved in a phosphate buffered saline, and thus a stimulating solution (concentration of acetylcholine: 50 $\mu$g/$\mu$L) was prepared.

To a hind leg of the mouse under the general anesthesia, 10 $\mu$L of a phosphate buffered saline containing no acetylcholine or 10 $\mu$L of the stimulating solution was hypodermically injected.

Two minutes after the injection, a skin sample of the hind leg of the mouse was taken, and the skin sample was embedded in an OCT compound (Lab-Tek Products, Illinois, USA).

From the OCT compound, a section having a thickness of 5 $\mu$m was prepared, and the section was stained in accordance with a known fluorescent staining method.

Primary antibodies used in the fluorescent staining method were anti-phospho-mTOR (ser2448) (1 : 50, Cell Signaling Technology, Tokyo, Japan) and anti-smooth muscle actin (SMA) (1 : 1000, DAKO, Tokyo, Japan).

Note that anti-phospho-mTOR is an antibody which recognizes a phosphorylated state of 2448th serine from an amino terminal of a mTOR protein, in other words, an antibody which recognizes an activated mTOR protein.

Moreover, a secondary antibody used in the fluorescent staining method was a commercially available secondary antibody (Invitrogen, Carlsbad, CA) with which Alexa Fluor 488 or Alexa Fluor 555 was linked.

A fluorescent-stained image was observed with use of BZ-8000 microscope (Keyence).

(a) through (d) of Fig. 1 show stained results in a case where the phosphate buffered saline containing no acetylcholine was used, and (a) through (d) of Fig. 2 show stained results in a case where the phosphate buffered saline containing acetylcholine was used.

More specifically, each of (a) of Fig. 1 and (a) of Fig. 2 shows a stained result of a mTOR, each of (b) of Fig. 1 and (b) of Fig. 2 shows a stained result of smooth muscle actin, each of (c) of Fig. 1 and (c) of Fig. 2 shows a stained result of Hoechst staining, and each of (d) of Fig. 1 and (d) of Fig. 2 shows an image obtained by superimposing the stained results shown in (a) through (c) of Fig. 1 or stained results shown in (a) through (c) of Fig. 2.

As shown in (a) of Fig. 1 and (a) of Fig. 2, in a case where perspiration was facilitated with use of acetylcholine, it was clarified that a stained image of the activated mTOR became vivid. That is, it was clarified that the mTOR was activated by facilitating perspiration with use of acetylcholine.

This (i) suggests that the mTOR plays an important role in adjusting perspiration and (ii) shows that perspiration can be inhibited by interfering with activity of the mTOR.

<2. Influence on skin exerted by administration of mTOR inhibitor>

An influence on a skin exerted by a mTOR inhibitor was observed.

Specifically, a mTOR inhibitor (everolimus (Afinitor (Registered Trademark)) was orally administered to a human by 10 mg per day, and a symptom appeared on the skin was observed.

Each of (a) and (b) of Fig. 3 is a photograph which shows a symptom appeared on the skin. Specifically, (a) of Fig. 3 is a photograph showing a state of the skin before the mTOR inhibitor is administered, and (b) of Fig. 3 is a photograph showing a state of the skin after the mTOR inhibitor is administered.

As a result of observing a symptom appeared on the skin, the symptom was dry skin which appeared because perspiration was inhibited.

That is, this result shows that perspiration can be inhibited by interfering with an activity of the mTOR.

<3. Influence on perspiration exerted by administration of mTOR inhibitor>

To a patient of renal angiomyolipoma in consequence of tuberous sclerosis, the mTOR inhibitor (everolimus (Afinitor (Registered Trademark))) was orally administered by 10 mg per day for approximately four months, and then the oral administration of the mTOR inhibitor was withdrawn.

During the period, after the oral administration of the mTOR inhibitor was started, an amount of sweating on a normal skin of the patient was measured with time and an amount of sweating on a skin of the patient on which a symptom of vitiligo appeared was measured with time.

The amount of sweating was measured with use of a local perspiration meter (quantitative axon reflex sweat amount measuring device) manufactured by Skinos Giken, and a specific measuring method was in conformity to a protocol attached to the device.

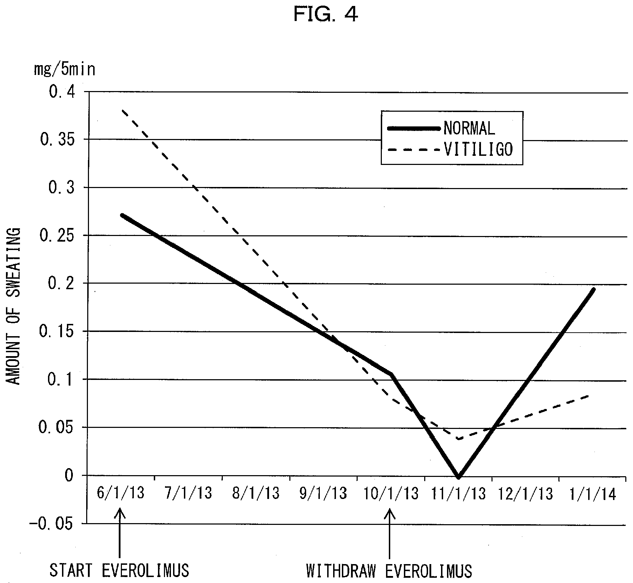

Fig. 4 shows change in amount of sweating. As shown in Fig. 4, the amount of sweating on the normal skin and the amount of sweating on the skin showing the symptom of vitiligo (mg/5 min) were reduced by administration of the mTOR inhibitor. Further, the amounts of sweating were restored by withdrawal of administration.

That is, the test clarified that the mTOR inhibitor had an effect of inhibiting an amount of sweating.

<4. Influence on perspiration by administration (external application, single administration) of mTOR inhibitor>

In this test, it was confirmed, in an animal experiment using mice, that the mTOR inhibitor had an effect of inhibiting perspiration. The following description will discuss a specific test method and test results.

Each of a gel containing no mTOR inhibitor, a gel containing 0.4% by weight of the mTOR inhibitor (specifically, rapamycin), and a gel containing 0.8% by weight of the mTOR inhibitor (specifically, rapamycin) was applied once by 0.2 g to a plantar part of a hind leg of the mouse which was a 11 to 12 week-old C57BL6 mouse.

Note that components other than the mTOR inhibitor in 1 g of the gel were 16 mg of Carbopol (Registered Trademark) 934NF, 490 mg of water, 480 mg to 490 mg of ethanol, and 6 mg of trishydroxymethylaminomethane.

More specifically, a composition of the gel (total weight: 100 g) containing 0.4% by weight of rapamycin was as follows: 0.4 g of rapamycin, 48.4 g of dehydrated ethanol, 43 g of injectable water, 1.6 g of Carbopol (Registered Trademark) 934NF, and 6.6 g of a trometamol solution (1 : 10). Moreover, a composition of the gel (total weight: 100 g) containing 0.8% by weight of rapamycin was as follows: 0.8 g of rapamycin, 48 g of dehydrated ethanol, 43 g of injectable water, 1.6 g of Carbopol (Registered Trademark) 934NF, and 6.6 g of a trometamol solution (1 : 10).

General anesthesia was applied to the mouse by intraabdominally injecting Domitor, Betorufaru, and Dormicum to the mouse 60 minutes after the application of the gel, and acetylcholine serving as a sudorific stimulus was administered to the mouse.

Next, with a known iodine-starch method, change in amount of sweating was measured.

First, mineral oil in which an iodine ethanol solution and starch were dissolved was applied to a plantar part of a hind leg of the mouse.

After that, pilocarpine hydrochloride (50 $\mu$g/20 $\mu$L/individual) was hypodermically injected to the plantar part of the hind leg of the mouse.

The plantar part of the hind leg of the mouse was observed five minutes after the injection, and the number of black dots appeared on the plantar part of the hind leg (more specifically, sweat glands existing in a paw pad of the plantar part) was counted. Note that the black dots are parts which are positive in the iodine-starch reaction. Moreover, statistical analysis was carried out with use of t-test.

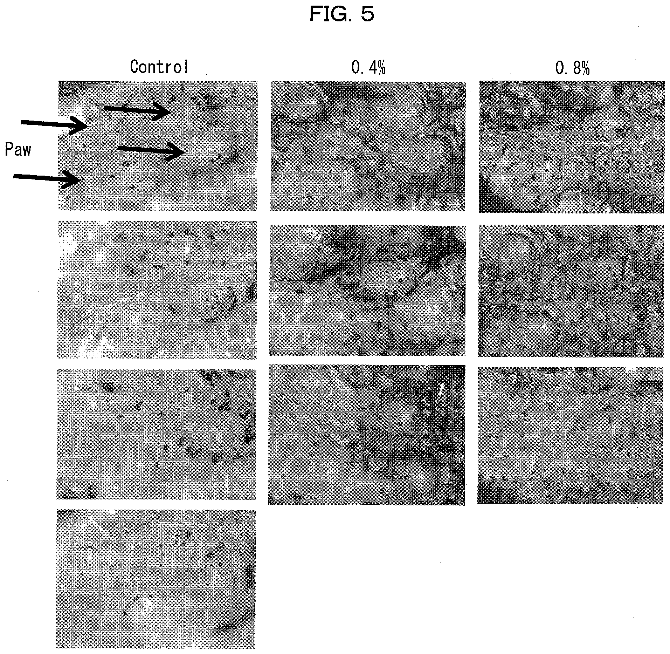

Fig. 5 is a photograph showing the plantar part of the hind leg of the mouse.

As shown in Fig. 5, in the case of the gel containing no mTOR inhibitor ("Control" in Fig. 5), many of the sweat glands existing in the paw pad were stained in black, and this clarified that perspiration was facilitated. On the other hand, in the case of the gel containing 0.4% by weight of the mTOR inhibitor ("0.4%" in Fig. 5) and the gel containing 0.8% by weight of the mTOR inhibitor ("0.8%" in Fig. 5), many of the sweat glands existing in the paw pad were not stained in black, and this clarified that perspiration was inhibited.

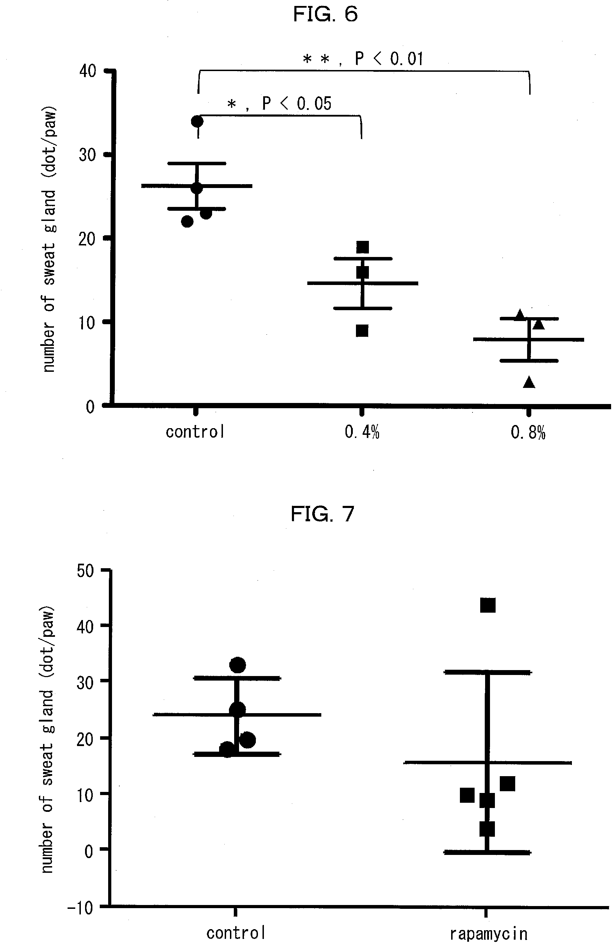

Fig. 6 is a graph showing the number of sweat glands which were stained in black. As shown in Fig. 6, in the case of the gel containing no mTOR inhibitor ("Control" in Fig. 6), perspiration was facilitated. On the other hand, in the cases of the gel containing 0.4% by weight of the mTOR inhibitor ("0.4%" in Fig. 6) and the gel containing 0.8% by weight of the mTOR inhibitor ("0.8%" in Fig. 6), it was clarified that perspiration was significantly inhibited.

Moreover, from Fig. 6, it was clarified that the increase in perspiration inhibitory effect had a dependence on concentration of the mTOR inhibitor.

<5. Influence on perspiration by administration of mTOR inhibitor (external application or internal application, sequential administration)>

<5-1. Regarding external application, sequential administration of mTOR inhibitor>

Each of a gel containing no mTOR inhibitor and a gel containing 0.4% by weight of the mTOR inhibitor (specifically, rapamycin) was applied once per day by 0.5 mg to a plantar part of a hind leg of the mouse which was a 11 to 12 week-old C57BL6 mouse. Then, this application was carried out for five consecutive days.

Note that components other than the mTOR inhibitor in the gel were identical with those in the formulation above described in <4. Influence on perspiration by administration (external application, single administration) of mTOR inhibitor>.

Then, 60 minutes after the last application, a sudorific stimulus was applied and also change in amount of sweating was measured in conformity to a known iodine-starch method. Note that the specific method for measuring an amount of sweating has been above described in <4. Influence on perspiration by administration (external application, single administration) of mTOR inhibitor>, and therefore the description of the method is omitted here, and only measurement results are described.

Fig. 7 is a graph showing the number of sweat glands which were stained in black. As shown in Fig. 7, in the case of the gel containing no mTOR inhibitor ("Control" in Fig. 7), perspiration was facilitated. On the other hand, in the case of the gel containing 0.4% by weight of the mTOR inhibitor ("Rapamycin" in Fig. 7), it was clarified that perspiration was inhibited.

<5-2. Regarding internal application, sequential administration of mTOR inhibitor>

Rapamycin was orally administered once per day to the mouse which was a 11 to 12 week-old C57BL6 mouse. Then, this oral administration was carried out for five consecutive days. Note that an amount of rapamycin which was administered to the mouse in one (1) oral administration was 2 mg per 1 kg of a body weight of the mouse.

Then, 60 minutes after the last oral administration, a sudorific stimulus was applied and also change in amount of sweating was measured in conformity to a known iodine-starch method (hereinafter, referred to as "test A").

Moreover, as another test, 10 days after the last oral administration, a sudorific stimulus was applied and also change in amount of sweating was measured in conformity to a known iodine-starch method (hereinafter, referred to as "test B").

The specific method for measuring an amount of sweating has been above described in <4. Influence on perspiration by administration (external application, single administration) of mTOR inhibitor>, and therefore the description of the method is omitted here, and only measurement results are described.

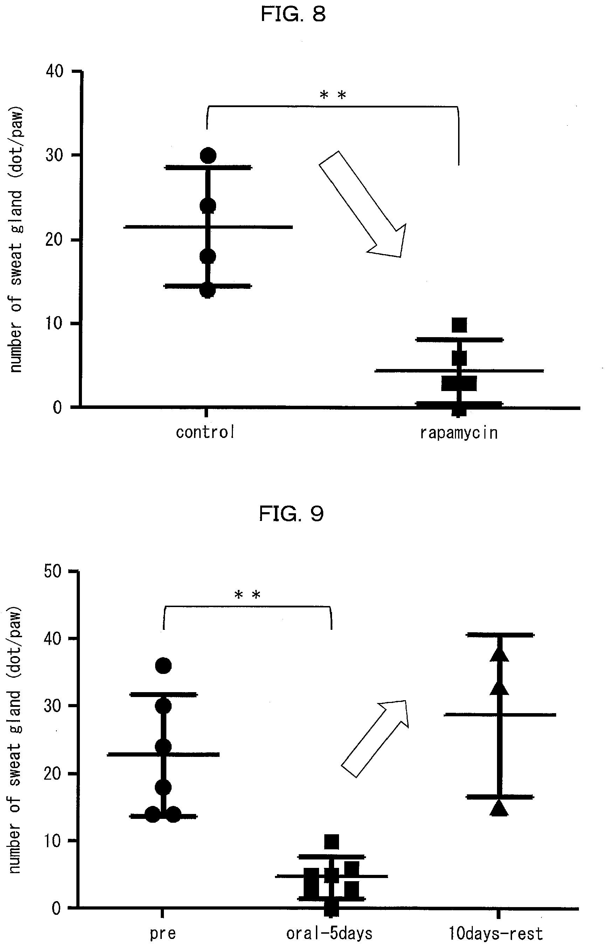

Fig. 8 is a graph showing the number of sweat glands which were stained in black in the test A. As shown in Fig. 8, in the mouse ("Control" in Fig. 8) to which the mTOR inhibitor was not orally administered, perspiration was facilitated. On the other hand, in the mouse ("Rapamycin" in Fig. 8) to which the mTOR inhibitor was orally administered, it was clarified that perspiration was inhibited.

Fig. 9 is a graph showing the number of sweat glands which were stained in black in the test B. As shown in Fig. 9, perspiration was inhibited in the mouse ("oral-5 days" in Fig. 9) to which the mTOR inhibitor was orally administered for five days, as compared with the mouse ("Pre" in Fig. 9) to which the mTOR inhibitor had not been administered yet. Moreover, it was clarified that the amount of sweating of the mouse ("10 days-rest" in Fig. 9) which was 10 days after withdrawal of oral administration of the mTOR inhibitor was restored to the amount before the mTOR inhibitor was orally administered.

That is, it was clarified that the perspiration inhibitory effect was a transient effect.

<6. Clinical test for investigating effect on amount of sweating by gel preparation containing rapamycin>

This test was carried out with approval obtained as a result of deliberation by Ethical Review Board of Osaka University.

<6-1. Effect of gel preparation containing rapamycin on amount of sweating (healthy subject)>

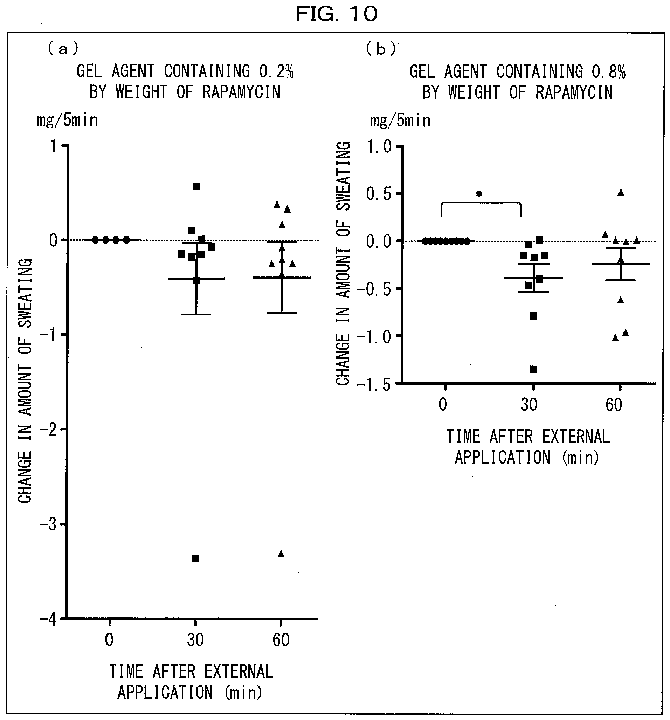

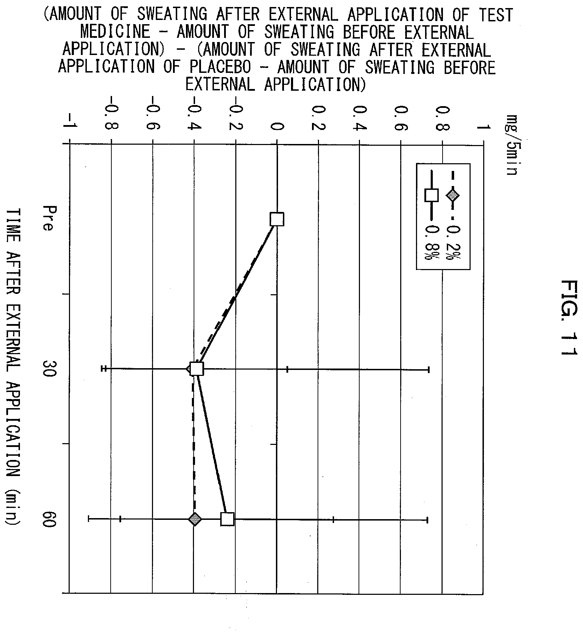

For each of nine healthy individuals, a gel preparation containing 0.2% by weight of rapamycin was externally applied to one palm (1 g/300 cm$^2$/application) and only a base material (i.e., a gel preparation (placebo) containing no rapamycin) was externally applied to the other palm, and an amount of sweating was measured before external application, 30 minutes after external application, and 60 minutes after external application, with a hermetic-ventilatory capsule method (Skinos Giken measuring device). Then, a difference in decreased amount of sweating between the part at which the gel preparation containing rapamycin was externally applied and the part at which the placebo was externally applied was calculated at each of the timings, and the difference was regarded as an effective decreased amount of sweating (Fig. 10, Fig. 11). Moreover, an experiment was carried out which was substantially identical with the above described one, except that, for each of another nine healthy individuals who were different from the healthy individuals to whom the gel preparation containing 0.2% by weight of rapamycin was applied, a gel preparation containing 0.8% by weight of rapamycin was externally applied to one palm (1 g/300 cm$^2$/application) and only a base material was externally applied to the other palm. Then, a difference in decreased amount of sweating between the part at which the gel preparation containing rapamycin was externally applied and the part at which the placebo was externally applied was calculated at each of the timings, and the difference was regarded as an effective decreased amount of sweating (Fig. 10, Fig. 11). Note that the statistical analysis was carried out with use of a t-test.

Specifically, a changed amount of sweating (also referred to as "decreased amount of sweating") was calculated with use of the following formula:

Changed amount of sweating (mg/5 min) = (amount of sweating predetermined time period after external application of gel preparation containing rapamycin - amount of sweating before external application) - (amount of sweating predetermined time period after external application of placebo - amount of sweating before external application)

Note that components other than the mTOR inhibitor (specifically, rapamycin) in 1 g of the gel were identical with those of the formulation above described in <4. Influence on perspiration by administration (external application, single administration) of mTOR inhibitor>.

Fig. 10 shows graphs indicating perspiration inhibitory effects with respect to healthy individuals by the rapamycin external medicine (specifically, gel preparation containing rapamycin) at different concentrations and different times. (a) of Fig. 10 shows results in a case where the gel preparation containing 0.2% by weight of rapamycin was applied, and (b) of Fig. 10 shows results in a case where the gel preparation containing 0.8% by weight of rapamycin was applied. As shown in Fig. 10, it was clarified that the amount of sweating decreased 30 minutes after the external application of the gel preparation containing rapamycin, as compared with the case before the gel preparation containing rapamycin was externally applied. It was also clarified that, in a case where the gel preparation containing 0.8% by weight of rapamycin was applied, the amount of sweating significantly decreased 30 minutes after the external application, as compared with the case before the external application (P < 0.05). Moreover, the decreased amount of sweating which was 60 minutes after the external application was approximately equal to the decreased amount of sweating which was 30 minutes after the external application.

Fig. 11 is a graph showing change with time of the amount of sweating after the rapamycin external medicine (specifically, gel preparation containing rapamycin) was applied. As shown in Fig. 11, it was clarified that the amount of sweating mostly decreased 30 minutes after the external application of the gel preparation containing rapamycin. Moreover, in a case where the gel preparation containing 0.8% by weight of rapamycin was applied, the decreased amount of sweating which was 60 minutes after the external application was approximately equal to the decreased amount of sweating which was 30 minutes after the external application.

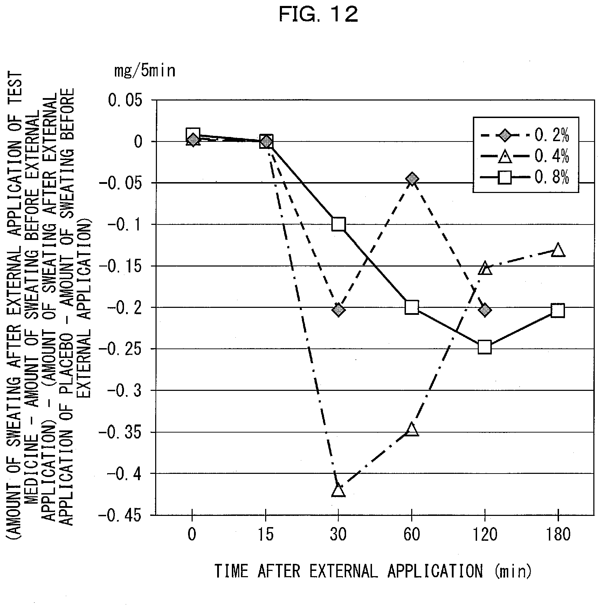

Next, for one (1) healthy individual, a gel preparation containing rapamycin by 0.2% by weight, 0.4% by weight, or 0.8% by weight was externally applied to one palm (1 g/300 cm$^2$/application) and only a base material (i.e., a gel preparation (placebo) containing no rapamycin) was externally applied to the other palm, and an amount of sweating was measured before external application, 15 minutes after external application, 30 minutes after external application, 60 minutes after external application, 120 minutes after external application, and 180 minutes after external application (in the test in which the gel preparation containing 0.2% by weight of rapamycin was used, before external application, 15 minutes after external application, 30 minutes after external application, 60 minutes after external application, and 120 minutes after external application), with a hermetic-ventilatory capsule method (Skinos Giken measuring device). Then, a difference in decreased amount of sweating between the part at which the gel preparation containing rapamycin was externally applied and the part at which the placebo was externally applied was calculated at each of the timings, and the difference was regarded as an effective decreased amount of sweating (Fig. 12). In this test, three tests were carried out on one (1) subject with use of respective three gel preparations containing different concentrations of rapamycin. The three tests were carried out at intervals of one week or more.

Fig. 12 is a graph showing change with time of the amount of sweating after the rapamycin external medicine (specifically, gel preparation containing rapamycin) was applied. As shown in Fig. 12, in cases where the gel preparation containing 0.2% by weight of rapamycin and the gel preparation containing 0.4% by weight of rapamycin were applied, it was clarified that the amount of sweating mostly decreased 30 minutes after the external application of the gel preparation containing rapamycin. Meanwhile, in a case where the gel preparation containing 0.8% by weight of rapamycin was applied, it was clarified that the amount of sweating mostly decreased 120 minutes after the external application of the gel preparation containing rapamycin. The decreased amount of sweating after the gel preparation containing rapamycin was externally applied was largest in the case where the gel preparation containing 0.4% by weight of rapamycin was applied. Note that stability and persistence in effect were better in the case of the gel preparation containing 0.8% by weight of rapamycin. The perspiration inhibitory effect was confirmed also 120 minutes after the application in each of cases of all the external medicines having the respective different concentrations. In the cases of the external medicines having concentrations of 0.4% by weight and 0.8% by weight, the perspiration inhibitory effect was confirmed even 180 minutes after the application.

<6-2. Effect of gel preparation containing rapamycin on amount of sweating (hyperhidrosis patient subject)>

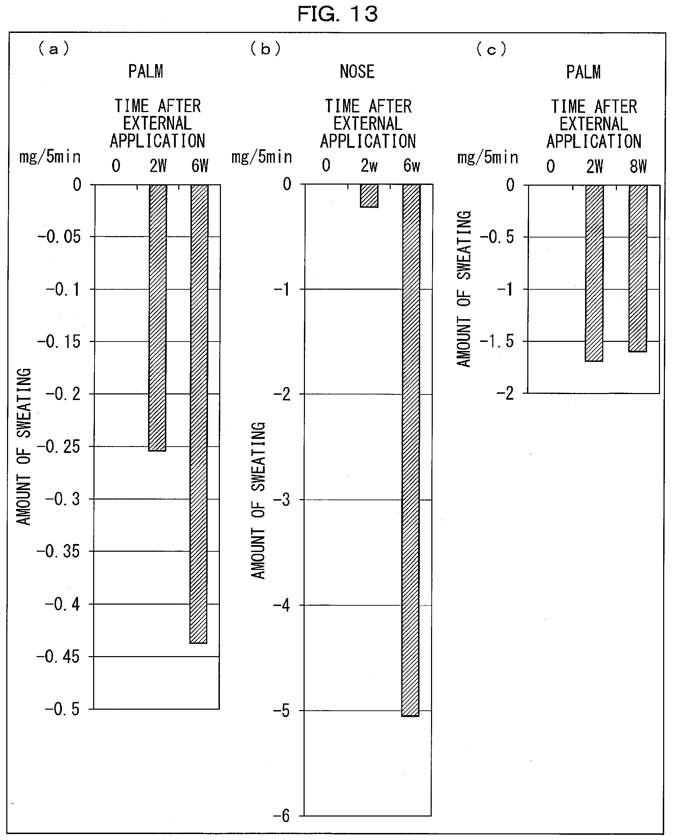

For each of three hyperhidrosis patients (subjects 1 through 3), the gel preparation containing rapamycin was applied to one of symmetric subject parts (palm and nose) by 0.2% by weight (1 g/300 cm$^2$/application) and only a base material (i.e., a gel preparation (placebo) containing no rapamycin) was externally applied to the other of the symmetric subject parts twice a day for 6 weeks as a double blind test. Medical examinations of each of the patients were carried out before the external application, 2 weeks after the external application started, and 6 weeks after the external application started. At each of the medical examinations, an amount of sweating before the external application and an amount of sweating 30 minutes after the external application were measured with the hermetic-ventilatory capsule method (by use of Skinos Giken measuring device), and a difference in decreased amount of sweating between the part at which the gel preparation containing rapamycin was externally applied and the part at which the placebo was externally applied was calculated, and the difference thus obtained was regarded as an effective decreased amount of sweating (Fig. 13). Note that the statistical analysis was carried out with use of a t-test.
Fig. 13 is a graph showing perspiration inhibitory effects exerted by the rapamycin external medicine (specifically, the gel preparation containing rapamycin) with respect to the hyperhidrosis patients. (a) of Fig. 13 shows a result in a case where the gel preparation containing rapamycin was applied to a palm of the subject 1, and a vertical axis of the graph indicates decreased amounts of perspiration at timings of before external application, 2 weeks after external application, and 6 weeks after external application. (b) of Fig. 13 shows a result in a case where the gel preparation containing rapamycin was applied to a nose of the subject 2, and a vertical axis of the graph indicates decreased amounts of perspiration at timings of before external application, 2 weeks after external application, and 6 weeks after external application. (c) of Fig. 13 shows a result in a case where the gel preparation containing rapamycin was applied to a palm of the subject 3, and a vertical axis of the graph indicates decreased amounts of perspiration at timings of before external application, 2 weeks after external application, and 8 weeks after external application.
The "decreased amount of sweating" was, specifically, calculated based on the following formula:

$$\text{Decreased amount of sweating (mg/5 min)} = (\text{amount of sweating 30 minutes after external application of gel preparation containing rapamycin} - \text{amount of sweating before external application}) - (\text{amount of sweating 30 minutes after external application of placebo} - \text{amount of sweating before external application})$$

As shown in Fig. 13, also for the hyperhidrosis patients, the perspiration inhibitory effect was brought about by the external application of the gel preparation containing rapamycin. Further, by the sequential administration of 6 weeks, the continuous and notable perspiration inhibitory effect was brought about.

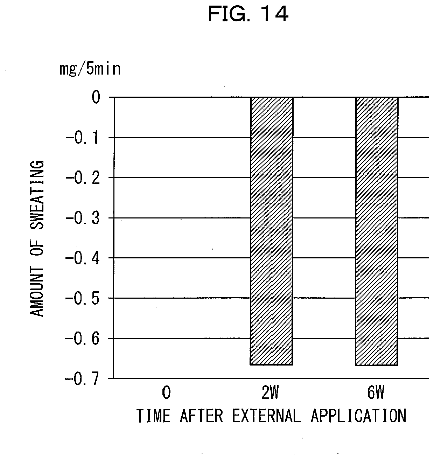

Fig. 14 is a graph showing a perspiration inhibitory effect of a rapamycin external medicine (specifically, the gel preparation containing rapamycin) with respect to a hyperhidrosis patient. Fig. 14 shows average values of decreased amount of sweating on the palms of the subjects 1 through 3 at timings of before external application, 2 weeks after external application, and 6 weeks after external application (for the subject 3, 8 weeks after external application). Table 1 shows decreased amounts of perspiration in the subjects 1 through 3 at timings of before external application, 2 weeks after external application, and 6 weeks after external application (for the subject 3, 8 weeks after external application).

As shown in Fig. 14, also for the hyperhidrosis patients, the perspiration inhibitory effect was brought about by the external application of the gel preparation containing rapamycin.

<6-3. Safety>

In the test with respect to the healthy individuals and the test with respect to the hyperhidrosis patients, no side effects were confirmed. As such, no problem in terms of safety was seen.

Industrial Applicability

The present invention is applicable to the field in which perspiration needs to be inhibited. For example, the present invention can be used to treat hyperhidrosis, miliaria, eccrine hidrocystoma, or dysidrosis (pompholyx).

**Claims**

1.    An antiperspirant comprising a mTOR inhibitor.

2.    The antiperspirant as set forth in claim 1, wherein:

    the mTOR inhibitor is rapamycin or a rapamycin derivative.

3.    The antiperspirant as set forth in claim 2, wherein:

    the rapamycin derivative is everolimus, temsirolimus, ridaforolimus, or zotarolimus.

4.    The antiperspirant as set forth in any one of claims 1 through 3, wherein:

    said antiperspirant is an external medicine, an internal medicine, or an injectable medicine.

5.    The antiperspirant as set forth in claim 4, wherein:

    said antiperspirant is the external medicine.

6.    The antiperspirant as set forth in any one of claims 1 through 5, wherein:

    said antiperspirant is used to treat hyperhidrosis, miliaria, eccrine hidrocystoma, or dysidrosis.

7.    An agent for treating hyperhidrosis comprising a mTOR inhibitor.

## FIG. 1

Ach (−)

(a)　　　　　　　　　　(b)

(c)　　　　　　　　　　(d)

FIG. 2

# FIG. 3

（a）

（b）

FIG. 4

FIG. 5

FIG. 6

FIG. 7

FIG. 8

FIG. 9

FIG. 10

(a) GEL AGENT CONTAINING 0.2% BY WEIGHT OF RAPAMYCIN

(b) GEL AGENT CONTAINING 0.8% BY WEIGHT OF RAPAMYCIN

FIG. 11

TIME AFTER EXTERNAL APPLICATION (min)

FIG. 12

## FIG. 13

FIG. 14

## INTERNATIONAL SEARCH REPORT

| International application No. |
|---|
| PCT/JP2015/069081 |

A. CLASSIFICATION OF SUBJECT MATTER
*A61K45/00*(2006.01)i, *A61K31/436*(2006.01)i, *A61K31/661*(2006.01)i,
*A61P17/00*(2006.01)i

According to International Patent Classification (IPC) or to both national classification and IPC

B. FIELDS SEARCHED

Minimum documentation searched (classification system followed by classification symbols)
A61K45/00, A61K31/436, A61K31/661, A61P17/00

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JMEDPlus/JST7580(JDreamIII), CAplus/REGISTRY/MEDLINE/EMBASE/
BIOSIS(STN), PubMed, CiNii, Ichushi WEB

C. DOCUMENTS CONSIDERED TO BE RELEVANT

| Category* | Citation of document, with indication, where appropriate, of the relevant passages | Relevant to claim No. |
|---|---|---|
| A | Takeshi NAKAHARA, "Other molecular target drug-associated dermatologic and mucosal problems", Journal of Clinical and Experimental Medicine, 26 May 2012 (26.05.2012), vol.241, no.8, pages 577 to 580 | 1-7 |
| A | Kazuhiko MATSUMOTO, "Cutaneous Toxicities Due to Small Molecules", Biotherapy, 2011.03, vol.25, no.2, pages 621 to 626 | 1-7 |
| A | Ichiro KATAYAMA, Hiroyuki MUROTA, Saki MATSUI, "Acetylcholine Yuhatsusei Hakkan no Seigyo Kiko no Kento", Nanjisei Jusho Genpatsusei Kyokusho Takansho no Byotai Kaiseki Oyobi Chiryo Shishin no Kakuritsu Heisei 23 Nendo Sokatsu·Buntan Kenkyu Hokokusho, 2012, pages 14 to 18 | 1-7 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 August 2015 (27.08.15) | 08 September 2015 (08.09.15) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

| International application No. |
|---|
| PCT/JP2015/069081 |

C (Continuation).   DOCUMENTS CONSIDERED TO BE RELEVANT

| Category* | Citation of document, with indication, where appropriate, of the relevant passages | Relevant to claim No. |
|---|---|---|
| A | Ichiro KATAYAMA, "Kessetsusei Kokasho no Hakuhan ni Taisuru Rapamycin Gel Gaiyo Rinsho Shiken", Shinkei Hifu Shokogun ni Kansuru Chosa Kenkyu Heisei 25 Nendo Sokatsu·Buntan Kenkyu Hokokusho, 31 March 2014 (31.03.2014), pages 105 to 108 | 1-7 |
| A | Ichiro KATAYAMA, "Kessetsusei Kokasho no Kekkan Sen'ishu ni Taisuru Rapamycin Gaiyo Chiryo no Yukosei", Shinkei Hifu Shokogun ni Kansuru Chosa Kenkyu Heisei 24 Nendo Sokatsu· Buntan Kenkyu Hokokusho, 29 March 2013 (29.03. 2013), pages 101 to 104 | 1-7 |

Form PCT/ISA/210 (continuation of second sheet) (July 2009)

| **INTERNATIONAL SEARCH REPORT** | International application No. |
| --- | --- |
| | PCT/JP2015/069081 |

Claim 1 relates to an antiperspirant agent containing, as an active ingredient, a compound that is defined by a desired property "a mTOR inhibitor".

Claim 1 includes all of compounds having the property. However, those compounds which are disclosed in the meaning within PCT Article 5 are just some of the claimed compounds. Therefore, this claim is not supported by the disclosure of the description in the meaning within PCT Article 6.

Furthermore, with respect to the "mTOR inhibitor", even though the common technical knowledge at the time of filing the present application is taken into consideration, it is impossible to specify the scope of the compound having the property. Therefore, claim 1 does not comply with the requirement of clarity under PCT Article 6, either.

Such being the case, the search was carried out on the relation between mTOR and perspiration and was also carried out on antiperspirant agents respectively containing, as active ingredients, the compounds which are described specifically in the description and are specified in claims 2 and 3.

Form PCT/ISA/210 (extra sheet) (July 2009)

**EP 3 162 380 A1**

**REFERENCES CITED IN THE DESCRIPTION**

**Non-patent literature cited in the description**

- Guideline of The Japanese Dermatological Association. *The Japanese journal of dermatology,* 2010, vol. 120 (8), 1607-1625